# EUROPEAN PATENT APPLICATION

(11) **EP 3 216 446 A1**
(43) Date of publication of application: **13.09.2017**
(21) Application number: 17156673.0
(22) Date of filing: 01.05.2013
(51) Int. Cl.: A61K 31/137, A61K 31/4178, A61P 13/00

(54) **METHODS FOR THE TREATMENT OF OVERACTIVE BLADDER**

(30) Priority: 01.05.2012 US 201261641290 P
(62) Divisional of application: 13724664.1
(71) Applicant: TheraVida, Inc., San Mateo CA 94402 (US)
(72) Inventor: PABORJI, Mehdi, Cupertino, CA California 95014 (US); FLUGEL, Roger, S., Redwood City, CA California 94061 (US); DUCHIN, Kenneth, L., Delray Beach, FL Florida 33446 (US)
(74) Representative: Viering, Jentschura & Partner mbB Patent- und Rechtsanwälte

(57) **Abstract**

Disclosed herein are methods of treating overactive bladder in a patient, the method comprising identifying a patient in need thereof; and administering to the patient a composition comprising tolterodine, or a pharmaceutically acceptable salt thereof, and pilocarpine, or a pharmaceutically acceptable salt thereof, wherein after the administration Cₘₐₓ for tolterodine is between 1.0-8.0 ng/mL.

## Description

### RELATED APPLICATIONS

The present application claims priority to the U.S. Provisional Application Serial No. 61/641,290, filed on May 1, 2012 by Paborji et al., and entitled "METHODS FOR THE TREATMENT OF OVERACTIVE BLADDER," the entire disclosure of which, including the drawings, is hereby incorporated by reference herein.

### FIELD OF THE INVENTION

The present invention is in the field of pharmaceutical compositions, and specifically in the field of compositions for the treatment of overactive bladder.

### BACKGROUND OF THE DISCLOSURE

Compositions and methods for the treatment of overactive bladder are known, where the compositions comprise a combination of tolterodine and pilocarpine. See for example, U.S. Patent 7,678,821 and U.S. Patent Application Publication No. 2011/0244051 A1, both of which are incorporated by reference herein in their entirety.

### SUMMARY OF THE INVENTION

Disclosed herein are methods of treating overactive bladder in a patient, the method comprising identifying a patient in need thereof; and administering to the patient a composition comprising tolterodine, or a pharmaceutically acceptable salt thereof, and pilocarpine, or a pharmaceutically acceptable salt thereof, wherein after the administration Cₘₐₓ for tolterodine is between approximately 1.0-8.0 ng/mL.

### BRlEF DESCRIPTION OF THE DRAWINGS

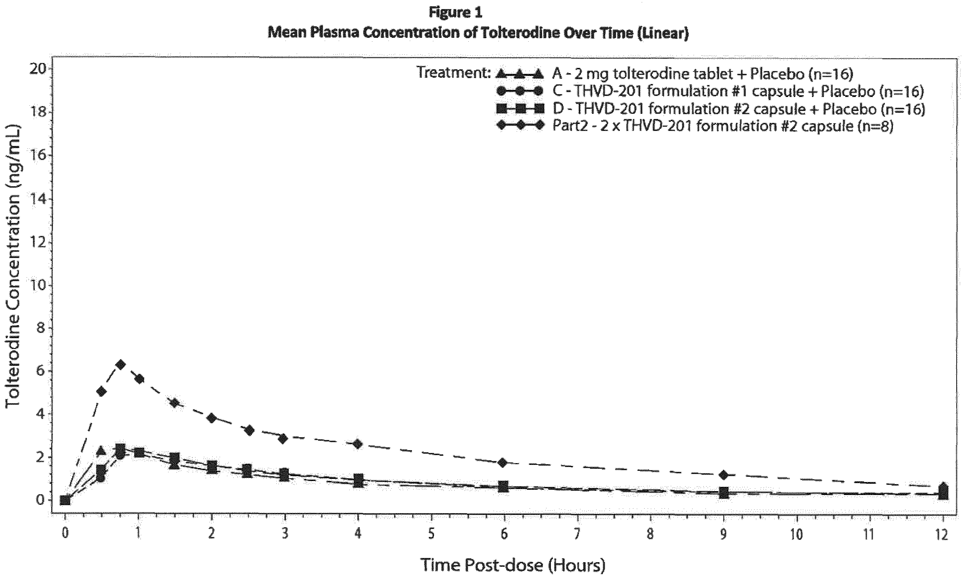

Figure 1 is a graph showing the mean plasma concentration of tolterodine over time collected in the studies disclosed herein.

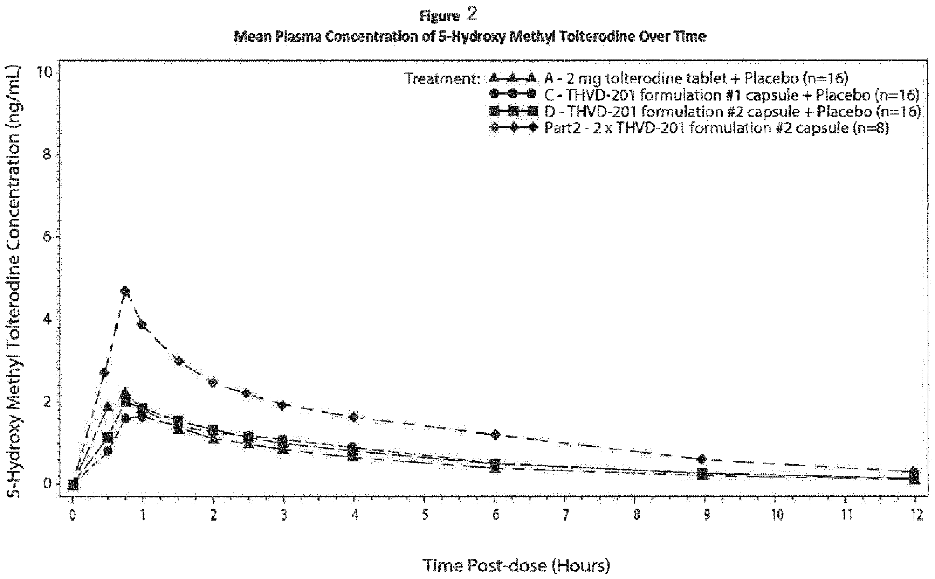

Figure 2 is a graph showing the mean plasma concentration of 5-hydroxy methyl tolterodine over time collected in the studies disclosed herein.

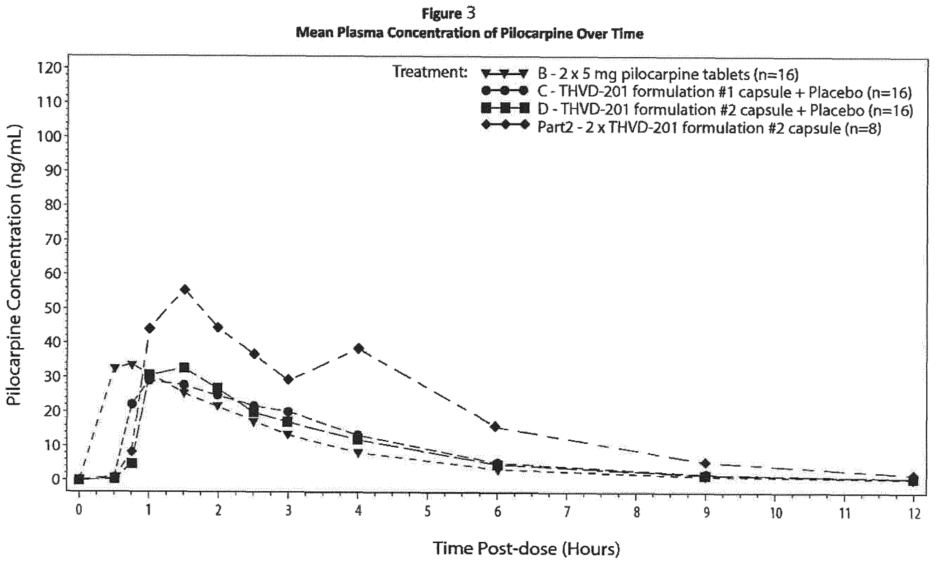

Figure 3 is a graph showing the mean plasma concentration of pilocarpine over time collected in the studies disclosed herein.

### DETAILED DESCRIPTION OF THE EMBODIMENTS

Disclosed herein are methods of treating overactive bladder in a patient, the method comprising identifying a patient in need thereof; and administering to the patient a composition comprising tolterodine, or a pharmaceutically acceptable salt thereof, and pilocarpine, or a pharmaceutically acceptable salt thereof, wherein after the administration Cₘₐₓ for tolterodine is between 1.0-8.0 ng/mL; Cₘₐₓ for pilocarpine is between 10-80 ng/mL; and Cₘₐₓ for 5-hydroxy methyl tolterodine is between 0.6-5.0 ng/mL.

The patient in need of the treatment is preferably a human having overactive bladder.

In some embodiments, the composition comprising tolterodine, or a pharmaceutically acceptable salt thereof, and pilocarpine, or a pharmaceutically acceptable salt thereof, is a composition as disclosed in the U.S. Patent Application Publication No. 2011/0244051 A1, entitled "PHARMACEUTICAL COMPOSITIONS FOR THE TREATMENT OF OVERACTIVE BLADDER," by Paborji et al., published October 6, 2011. The disclosure of this publication in its entirety, and specifically the disclosure of various compositions, for example those set forth in Paragraphs [0071]-[0080], is incorporated by reference herein.

In some embodiments, during the dosage interval, serum concentration of tolterodine fluctuates no more than 8.0 ng/mL. In some embodiments, during the dosage interval, serum concentration of pilocarpine fluctuates no more than 80 ng/mL. In some embodiments, during the dosage interval, serum concentration of 5-hydroxy methyl tolterodine fluctuates no more than 5.0 ng/mL.

By "dosage interval" it is meant the period of time between two consecutive administrations of the pharmaceutical composition during repeated administration. By "fluctuates no more than" a certain value, throughout this disclosure, it is meant that the serum concentration at its lowest level during the dosage interval subtracted from the serum concentration at its highest level during the dosage interval is less than the specified value.

In some embodiments, after the administration Cₘₐₓ for tolterodine is between 1.5-7.5 ng/mL. In other embodiments, after the administration Cₘₐₓ for tolterodine is between 1.5-6.0 ng/mL. In other embodiments, after the administration Cₘₐₓ for tolterodine is between 2.0-5.0 ng/mL. In other embodiment, after the administration Cₘₐₓ for tolterodine is between 2.0-4.0 ng/mL. In other embodiments, after the administration Cₘₐₓ for tolterodine is between 2.5-4.0 ng/mL. In other embodiments, after the administration Cₘₐₓ for tolterodine is between 2.5-3.5 ng/mL.

In some embodiments, after the administration Cₘₐₓ for pilocarpine is between 15-75 ng/mL. In other embodiments, after the administration Cₘₐₓ for pilocarpine is between 20-75 ng/mL. In other embodiments, after the administration Cₘₐₓ for pilocarpine is between 25-70 ng/mL. In other embodiments, after the administration Cₘₐₓ for pilocarpine is between 30-70 ng/mL. In other embodiments, after the administration Cₘₐₓ for pilocarpine is between 35-60 ng/mL. In other embodiments, after the administration Cₘₐₓ for pilocarpine is between 35-50 ng/mL. In other embodiments, after the administration Cₘₐₓ for pilocarpine is between 35-45 ng/mL.

In some embodiment, after the administration Cₘₐₓ for 5-hydroxy methyl tolterodine is between 1.0-5.0 ng/mL. In other embodiments, after the administration Cₘₐₓ for 5-hydroxy methyl tolterodine is between 1.5-4.5 ng/mL. In other embodiments, after the administration Cₘₐₓ for 5-hydroxy methyl tolterodine is between 1.5-4.0 ng/mL. In other embodiments, afrer the administration Cₘₐₓ for 5-hydroxy methyl tolterodine is between 2.0-4.0 ng/mL. In other embodiments, after the administration Cₘₐₓ for 5-hydroxy methyl tolterodine is between 2.0-3.0 ng/mL.

In some embodiment, the Cₘₐₓ for tolterodine is greater than 3.0 ng/mL. In some embodiments Cₘₐₓ for pilocarpine is greater than 40 ng/mL. In some embodiments, Cₘᵢₙ for tolterodine before the administration of the next dose is greater than 1 ng/mL. In some embodiment, Cₘᵢₙ for pilocarpine before the administration of the next dose is greater than 1 ng/mL. In some embodiments, Tₘₐₓ for tolterodine is between 0.5-2.0 hr. In some embodiments, Tₘₐₓ for pilocarpine is between 0.5-2.0 hr. In some embodiments, Tₘₐₓ for 5-hydroxy methyl tolterodine is between 0.5-2.0 hr.

The definition of the pharmacokinetic parameters Cₘₐₓ, Cₘᵢₙ, and Tₘₐₓ is well-known to those of skill in the art. Briefly, Cₘₐₓ is the maximum observed plasma concentration during the dosage interval. Cₘᵢₙ is the minimum observed plasma concentration during the dosage interval. Tₘₐₓ is the time period from the point of administration to maximum observed concentration.

### Example 1: Pharmacokinetic Study

The first part of the study was a randomized, double-blind, single center, single dose, five-period, five-treatment, crossover study in 18 healthy individuals. On days 1, 8, 15, 22 and 29 of part 1, subjects received one of the following treatments according to the following randomized treatment schedule:
- Treatment A: 2 mg tolterodine tablet + placebo capsule
- Treatment B: 2 x 5 mg pilocarpine tablets
- Treatment C: 2 mg tolterodine/11 mg pilocarpine Formulation #1 capsule + placebo capsule
- Treatment D: 2 mg tolterodine/11 mg pilocarpine Formulation #2 capsule + placebo capsule
- Treatment E: 2 x placebo capsules

Formulation #1 and Formulation #2 are formulations comprising beads of pilocarpine and heads of tolterodine. In Formulation #1, the pilocarpine beads release the pilocarpine after about 20 minutes after contact with acidic media, based on the in vitro dissolution data. In Formulation #2 the delay is 30 minutes.

Treatments were administered with 240 mL of room temperature tap water. In order to preserve the blinding, a placebo capsule was given with each of the treatments, except for Treatment B. Each subject received each treatment at least 7 days apart.

Part 2 of this study was an open label, single dose, one treatment study, in 9 healthy individuals who had completed Part 1 of the study, in which a higher dose (2 capsules) of Formulation #2 was administered in the same manner as in Part 1.

### Pharmacokinetics

Blood samples for determination of tolterodine, the tolterodine metabolite (5-hydroxy methyl tolterodine) and pilocarpine measurement were collected on Days 1, 8, 15, 22, 29 (Part 1) and Day 2A (Part 2) at pre-dose, 0.5, 0.75, 1, 1.5, 2, 2.5, 3, 4, 5, 9, and 12 hours post-dose. Blood was collected into vacutainer tubes containing fluoride oxalate and was immediately placed on ice and centrifuged under refrigeration within 30 min of collection. The plasma was divided into 2 aliquots in polypropylene plain tubes and then stored at -70 °C or below until analysis. Plasma samples were analyzed for concentrations of tolterodine, 5-hydroxy methyl tolterodine, and pilocarpine by validated methods.

The following key plasma pharmacokinetic parameters were measured for each individual in the study using standard procedures:
- Cₘₐₓ, maximum observed plasma concentration directly from the data.
- Tₘₐₓ, time to maximum observed concentration, taken directly from data.
- AUC₀₋₁, area under the plasma concentration versus time curve, calculated using the linear trapezoidal rule from time 0 to time t, where t is the last quantifiable concentration.
- t_{½}, apparent terminal half-life, calculated as t_{½} = ln(2)/k_{cl}, where ln(2) = 0.693 and k_{cl} is the terminal elimination rate constant, obtained from the slope of the line, filted by linear least squares regression through the terminal points of the logarithmic concentration-time profiles.
- AUC_{0-inf}, area under the plasma concentration versus time curve from zero to infinity, calculated as (AUC₀₋₁ + C₁/k_{cl}), where C₁ is the last quantifiable concentration.

The mean (± standard deviation (SDV)) and coefficient of variation of the above key plasma pharmacokinetic parameters by treatment for the 16 subjects in the pharmacokinetic analysis set are provided in Table 1.

**Table 1: Mean (CV%) of Key Pharmacokinetic Parameters**

| | Treatment | Tₘₐₓ | Cₘₐₓ | AUC_{0-dast} | t_{½} | AUC₀₋ₜᵢₙₗ |
|---|---|---|---|---|---|---|
| | | (hr) | (ng/ml.) | (hr*ng/mL) | (hr) | (hr*ng/mL) |
| Tolterodine | A | 0.63±0.18 | 3.14±2.75 | 8.68±10.63 | 2.14±0.66 | 7.23±5.80 |
| | (n=16) | (29%) | (88%) | (122%) | (31%) | (80%) |
| | C | 1.61±1.12 | 2.77±2.37 | 9.07±10.49 | 2.05±0.44 | 7.68±5.83 |
| | (n=16) | (69%) | (85%) | (116%) | (22%) | (76%) |
| | D | 1.05±0.84 | 2.93±2.93 | 9.60±11.07 | 2.01±0.54 | 8.11±6.22 |
| | (n=16) | (80%) | (68%) | (115%) | (27%) | (77%) |
| | Part 2 | 0.84±0.48 | 7.32±5.78 | 25.85±27.27 | 2.34±1.12 | 29.39±35.05 |
| | (n=8) | (57%) | (79%) | (106%) | (48%) | (119%) |
| 5-hydroxy methyl tolterodine | A | 0.67±0.18 | 2.22±1.08 | 6.60±3.20 | 3.01±0.66 | 7.50±2.94 |
| | (n=16) | (27%) | (49%) | (49%) | (22%) | (39%) |
| | C | 1.73±1.14 | 1.97±1.01 | 71.1±3.19 | 2.94±0.57 | 8.17±2.95 |
| | (n=16) | (65%) | (51%) | (45%) | (19%) | (36%) |
| | D | 1.07±0.84 | 2.12±0.90 | 7.29±3.28 | 3.00±0.62 | 8.32±2.93 |
| | (n=16) | (78%) | (42%) | (45%) | (21%) | (35%) |
| | Part 2 | 0.94±0.26 | 4.38±2.47 | 15.73±9.02 | 2.84±0.25 | 19.14±7.63 |
| | (n=8) | (28%) | (56%) | (57%) | (9%) | (40%) |
| Pilocarpine ne | A | 0.71±0.26 | 38.4±10.1 | 98.2±33.2 | 1.82±0.42 | 99.4±33.7 |
| | (n=16) | (37%) | (26%) | (34%) | (23%) | (34%) |
| | C | 1.63±0.88 | 38.8±21.1 | 106.7±47.4 | 1.76±0.34 | 108.5±48.2 |
| | (n=16) | (54%) | (54%) | (44%) | (19%) | (44%) |
| | D | 1.50±0.55 | 37.3±13.4 | 98.9±37.5 | 1.83±0.41 | 100.6±38.3 |
| | (n=16) | (37%) | (36%) | (38%) | (22%) | (38%) |
| | Part 2 | 1.75±0.96 | 66.7±19.3 | 223.8±83.9 | 1.79±0.21 | 228.6±88.2 |
| | (n-8) | (55%) | (29%) | (37%) | (12%) | (39%) |

### Salivary Flow

Stimulated salivary flow (SSF) and dry mouth (assessed using a "visual analog scale," or VAS) were determined at frequent intervals after administration of each treatment. Urine frequency, urine volume/void, and fluid volume consumed were also assessed for each treatment.

SSF was measured on Days 1, 8, 15, 22, and 29 (Part 1) and Day 2A (Part 2) 30 minutes prior to and at 1, 1.5, 2, 2.5, 3, 3.5, 6, 9, and 12 hours post-dose. SSF was measured in the following manner. At the specified time (approximately 15 minutes prior to a blood sample), each subject rinsed their mouth with approximately 60 mL of tap water, expectorating the water after rinsing. Ten minutes (±2 min) later each subject was told to swallow any saliva in his mouth and an accurately weighed 2.5x2.5 cm² square of parafilm was placed on each subject's tongue. Subjects chewed the parafilm for exactly 2 min, in a consistent manner, and after which any accumulated saliva and the chewed paraffin was expectorated into a pre-weighed container and the container reweighed. The subject may have expectorated saliva in the containers several times if there was excessive saliva.

The SSF values were tabulated at each time point and descriptive slatistics were generated by treatment, for measured values and changes from pre-dose baseline. Total weight of saliva over time was analyzed using analysis of variance, with Tukey's method of comparison between treatments. Dry mouth was tabulated at each time point and descriptive statistics were generated by treatment, for measured values and changes from pre-dose baseline. Urine frequency, urine volume/void and fluid volume consumed were tabulated and summarized by treatment.

Table 2 summarizes the mean (± standard deviation) of change over time in stimulated salivary flow from pre-dose baseline by treatment for the evaluated subjects in the pharmacodynamic analysis set.

**Table 2: Mean (SD) SSF Change (g) from Pre-dose Baseline**

| Treatment | Time Post-dose (Hours) | | | | | | | | | |
|---|---|---|---|---|---|---|---|---|---|---|
| | 1 | 1.5 | 2 | 2.5 | 3 | 3.5 | 4 | 6 | 9 | 12 |
| A | -0.3 | -0.47 | -0.42 | -0.30 | -0.28 | -0.07 | 0.05 | 0.21 | -0.06 | 0.27 |
| (n=16) | (0.73) | (0.67) | (0.80) | (0.61) | (0.68) | (0.65) | (0.61) | (0.50) | (0.75) | (0.79) |
| **B** | 4.55 | 3.23 | 2.33 | 1.26 | 1.07 | 0.93 | 0.64 | 0.32 | -0.20 | -0.08 |
| (n=16) | (1.95) | (1.89) | (1.32) | (0.85) | (0.61) | (0.52) | (0.71) | (0.54) | (0.63) | (0.59) |
| C | 1.90 | 0.95 | 0.81 | 0.71 | 0.60 | 0.60 | 0.51 | 0.31 | 0.14 | 0.13 |
| (n=17) | (1.30) | (1.11) | (0.93) | (0.90) | (0.88) | (0.70) | (0.84) | (0.83) | (0.83) | (0.82) |
| D | 1.40 | 1.08 | 0.96 | 0.76 | 0.76 | 0.90 | 0.61 | 0.52 | 0.17 | 0.47 |
| (n=17) | (1.32) | (0.76) | (0.69) | (0.71) | (0.82) | (0.62) | (0.69) | (0.69) | (0.72) | (0.38) |
| E | 0.31 | 0.17 | 0.11 | 0.14 | 0.21 | 0.36 | 0.46 | 0.30 | 0.18 | 0.27 |
| (n=17) | (0.70) | (0.74) | (0.47) | (0.48) | (0.44) | (0.61) | (0.64) | (0.51) | (0.49) | (0.57) |
| Part 2 | 0.21 | 0.80 | 0.81 | 0.44 | 0.40 | 0.84 | 1.18 | 0.78 | 0.14 | 0.35 |
| (n=8) | (0.82) | (1.80) | (1.20) | (1.00) | (0.70) | (1.04) | (0.64) | (1.05) | (0.99) | (1.11) |

The observed decrease in SSF following the administration of tolterodine was blunted by both Formulations #1 and #2 (Treatments C and D), with the SSF after each of these test formulations was slightly above the levels for placebo, but less than for pilocarpine alone (Treatment B). Doubling the dose of Formulation #2 (Treatment Part 2) did not appreciably alter the change from baseline in SSF or the degree of dry mouth (VAS) compared to the lower dose.

### Dry Mouth

Dry mouth was assessed on Days 1, 8, 15, 22, and 29 (Part 1) and Day 2A (Part 2) prior to and at 1, 2, 2.5, 3, 4, and 6 hours post-dose. For qualitative assessment of dry mouth, the "visual analog scale," or VAS, scale (0=not dry, 10=very dry, measured in centimeters) was used. Each subject was asked to mark a vertical line the VAS scale on how dry their mouth was at that moment.

VAS is a well-known method. In this method, subjects were shown a line scaled from 0 to 10 cm. Subjects were asked to rate the subjective criterion from 0-10 cm and make a mark on the line corresponding to their rating. For example, subjects were told that 0 cm on the line means no dry mouth at all and 10 cm on the line means extreme dry mouth. The subjects rated their extent of dry mouth on the line, Changes in the extent of dry mouth of a subject were measured using this technique throughout the treatment period.

Mean VAS scores of dry mouth over time for pilocarpine (Treatment B) were markedly lower at the 1 hour and 2 hour time points compared with each of the other treatments. Table 3, below, shows the data for the dry mouth assessment.

**Table 3: Summary of Change from Baseline of Dry Mouth Assessment by Treatment**

| | | Time Post-dose (Hours) | | | | | |
|---|---|---|---|---|---|---|---|
| Treatment | | 1 | 2 | 2.5 | 3 | 4 | 6 |
| A | Mean | 0.03 | 0.63 | 0.56 | 0.97 | 0.56 | 0.00 |
| (n=16) | SDV | 1.23 | 2.35 | 1.53 | 1.88 | 1.54 | 1.40 |
| B | Mean | -3.00 | -2.34 | -1.38 | -0.94 | 0.69 | -0.41 |
| (n=16) | SDV | 1.76 | 1.52 | 1.59 | 1.05 | 0.77 | 1.05 |
| C | Mean | -0.71 | -1.32 | -0.81 | -0.53 | -0.53 | -0.56 |
| (n=17) | SDV | 1.50 | 1.50 | 1.78 | 1.60 | 1.55 | 1.61 |
| D | Mean | -0.21 | -0.88 | -0.62 | -0.38 | -0.65 | -0.41 |
| (n=17) | SDV | 0.99 | 1.81 | 1.65 | 1.56 | 1.53 | 1.30 |
| E. | Mean | 0.06 | 0.29 | 0.38 | 0.62 | 0.71 | 0.21 |
| (n=17) | SDV | 1.09 | 1.23 | 1.40 | 1.67 | 1.49 | 1.10 |
| Part 2 | Mean | -0.06 | -0.13 | -0.31 | -0.13 | 0.69 | 0.50 |
| (n=8) | SDV | 1.15 | 1.64 | 1.31 | 0.99 | 0.92 | 0.85 |

### Further embodiments

1. A method of treating overactive bladder in a patient, the method comprising:
   identifying a patient in need thereof; and
   administering to the patient a composition comprising tolterodine, or a pharmaceutically acceptable salt thereof, and pilocarpine, or a pharmaceutically acceptable salt thereof,
   wherein after the administration:
      Tmax for tolterodine is between 0.5-2.0 hr;
      Tmax for pilocarpine is between 0.5-2.0 hr; and
      Tmax for 5-hydroxy methyl tolterodine is between 0.5-2.0 hr.
2. The method of embodiment 1, wherein during the dosage interval, serum concentration of tolterodine fluctuates no more than 8.0 ng/mL.
3. The method of embodiment 1, wherein during the dosage interval, serum concentration of pilocarpine fluctuates no more than 70 ng/mL.
4. The method of embodiment 1, wherein during the dosage interval, serum concentration of 5-hydroxy methyl tolterodine fluctuates no more than 5.0 ng/mL.
5. The method of embodiment 1, wherein the Cmax for tolterodine is greater than 3.0 ng/mL.
6. The method of embodiment 1, wherein Cₘₐₓ for pilocarpine is greater than 40 ng/mL.
7. The method of embodiment 1, wherein C_{miₙ} for tolterodine before the administration of a subsequent dose is greater than 1 ng/mL.
8. The method of embodiment 1, wherein C_{ₘiₙ} for pilocarpine before the administration of a subsequent dose is greater than 1 ng/mL.
9. The method of embodiment 1, Cₘₐₓ for tolterodine is between 1.0-8.0 ng/mL.
10. The method of embodiment 1, Cₘₐₓ for pilocarpine is between 10-80 ng/mL.
11. The method of embodiment 1, Cmax for 5-hydroxy methyl tolterodine is between 0.6-5.0 ng/mL.
12. A method of treating overactive bladder in a patient, the method comprising:
   identifying a patient in need thereof; and
   administering to the patient a composition comprising tolterodine, or a pharmaceutically acceptable salt thereof, and pilocarpine, or a pharmaceutically acceptable salt thereof,
   wherein after the administration Tₘₐₓ for tolterodine is between 0.5-2.0 nr.
13. The method of embodiment 12, wherein Cₘₐₓ for pilocarpine is between 10-80 ng/mL.
14. The method of embodiment 12, wherein Cₘₐₓ for 5-hydroxy methyl tolterodine is between 0.6-5.0 ng/mL.
15. The method of embodiment 12, wherein during the dosage interval, serum concentration of tolterodine fluctuates no more than 8.0 ng/mL.
16. The method of embodiment 12, wherein during the dosage interval, serum concentration of pilocarpine fluctuates no more than 70 ng/mL.
17. The method of embodiment 12, wherein the Cₘₐₓ for tolterodine is greater than 3.0 ng/mL.
18. The method of embodiment 12, wherein Cₘₐₓ for pilocarpine is greater than 40 ng/mL.
19. The method of embodiment 12, wherein Cₘiₙ for tolterodine before the administration of a subsequent dose is greater than 1 ng/mL.
20. The method of embodiment 12, wherein Cₘiₙ for pilocarpine before the administration of a subsequent dose is greater than 1 ng/mL.
21. The method of embodiment 12, C_{maX} for tolterodine is between 1.0-8.0 ng/mL.
22. The method of embodiment 12, Tₘₐₓ for pilocarpine is between 0.5-2.0 hr.

## Claims

1. Composition comprising tolterodine, or a pharmaceutically acceptable salt thereof, and pilocarpine, or a pharmaceutically acceptable salt thereof for use in a method of treating overactive bladder in a patient, the method comprising:
identifying a patient in need thereof; and
administering to the patient said composition,
wherein after the administration:
Cₘₐₓ for tolterodine is between 2.5-8.0 ng/mL.

2. The composition for use according to claim 1, wherein after the administration Cₘₐₓ for pilocarpine is between 30-80 ng/mL.

3. The composition for use according to claim 1 or 2, wherein after the administration Cₘₐₓ for 5-hydroxy methyl tolterodine is between 1.5-5.0 ng/mL.

4. The composition for use according to any one of claims 1-3, wherein after the administration Cmax for tolterodine is between 2.5-8.0 ng/mL; Cₘₐₓ for pilocarpine is between 30-80 ng/mL; and Cₘₐₓ for 5-hydroxy methyl tolterodine is between 1.5-5.0 ng/mL.

5. The composition for use according to any one of claims 1-4, wherein during the dosage interval, serum concentration of tolterodine fluctuates no more than 8.0 ng/mL.

6. The composition for use according to any one of claims 1-5, wherein during the dosage interval, serum concentration of pilocarpine fluctuates no more than 70 ng/mL.

7. The composition for use according to any one of claims 3-6, wherein during the dosage interval, serum concentration of 5-hydroxy methyl tolterodine fluctuates no more than 5.0 ng/mL.

8. The composition for use according to any one of claims 1-7, wherein the Cₘₐₓ for tolterodine is greater than 3.0 ng/mL.

9. The composition for use according to any one of claims 1-8, wherein Cₘₐₓ for pilocarpine is greater than 40 ng/mL.

10. The composition for use according to any one of claims 1-9, wherein Cₘᵢₙ for tolterodine before the administration of the next dose is greater than 1 ng/mL.

11. The composition for use according to any one of claims 1-10, wherein Cₘᵢₙ for pilocarpine before the administration of the next dose is greater than 1 ng/mL.

12. The composition for use according to any one of claim 1-11, Tₘₐₓ for tolterodine is between 0.5-2.0 hr.

13. The composition for use according to any one of claims 1-12, Tₘₐₓ for pilocarpine is between 0.5-2.0 hr.

14. The composition for use according to any one of claims 3-13, Tₘₐₓ for 5-hydroxy methyl tolterodine is between 0.5-2.0 hr.
